Europäisches Patentamt

European Patent Office

Office européen des brevets

(11) Veröffentlichungsnummer: **0 492 305 A1**

# (12) EUROPÄISCHE PATENTANMELDUNG

(21) Anmeldenummer: **91121348.6**

(22) Anmeldetag: **12.12.91**

(51) Int. Cl.⁵: **A61K 35/78**

(30) Priorität: **20.12.90 DE 4040961**

(43) Veröffentlichungstag der Anmeldung:
**01.07.92 Patentblatt 92/27**

(84) Benannte Vertragsstaaten:
**AT BE CH DE DK ES FR GB GR IT LI LU MC NL SE**

(71) Anmelder: **Dr. Willmar Schwabe GmbH & Co.**
**Dr. Willmar-Schwabe-Strasse 4**
**W-7500 Karlsruhe 41(DE)**

(72) Erfinder: **Schwabe, Klaus-Peter, Dr.**
**Strählerweg 113**
**W-7500 Karlsruhe 41(DE)**

(74) Vertreter: **Bunke, Holger, Dr.rer.nat.**
**Dipl.-Chem. et al**
**Patentanwälte Prinz, Leiser, Bunke & Partner**
**Manzingerweg 7**
**W-8000 München 60(DE)**

(54) **Sabal-Extrakt, Verfahren zu seiner Herstellung und Verwendung.**

(57) Sabal-Extrakt aus Sabal serrulatum (Serenoa repens) mit hohem Sabalesteranteil, gekennzeichnet durch einen Gehalt an Ethylestern der Caprin-, Laurin- und Myristinsäure von zusammen 12 bis 18 Gew.-%, vorzugsweise standardisiert auf 15 Gew.-%. Der an Sabalestern angereicherte, ölige Trockenextrakt besitzt im Vergleich zu herkömmlichen ethanolischen Extrakten und Tinkturen, die Sabalestergehalte von weniger als 5 % aufweisen, signifikant höhere antiandrogene und antiexsudative Wirksamkeit und kann deshalb zur Herstellung von Arzneimitteln oder selbst als Arzneimittel, gegebenenfalls zusammen mit üblichen Hilfs- und Zusatzstoffen, verwendet werden. Das erfindungsgemäße Verfahren zur Herstellung des Sabal-Extraktes ist dadurch gekennzeichnet, daß das Ethanol-Mazerat solange auf einem pH-Wert von etwa 3 gehalten wird, bis der Sabalestergehalt 12 bis 18 Gew.-%, bezogen auf den Trockenextrakt, beträgt.

EP 0 492 305 A1

Die Erfindung betrifft die Bereitstellung eines standardisierbaren Extraktes aus den Früchten der Zwergpalme Sabal serrulatum (Serenoa repens) mit hohem Gehalt an "Sabalestern", d.h. Ethylestern der Caprin-, Laurin- und Myristinsäure.

Sogenannte Lipidextrakte der Sabal-Droge werden traditionell zur Therapie prostatischer Beschwerden eingesetzt. Als lipophile Lösungsmittel kommen hierbei in Betracht verschiedene Alkohole wie z.B. Ethanol, Ketone wie z.B. Aceton/Butanon, Ester wie z.B. Ethylacetat, Kohlenwasserstoffe wie z.B. Hexan und halogenierte Kohlenwasserstoffe wie z.B. Dichlormethan (vgl. Z. f. Phytotherapie 10, 71-76 (1989) und EP-A-0 068 055).

Ethanolische Extrakte mit reinem Ethanol oder Ethanol/Wasser-Mischungen (Tinkturen) besitzen nun in ihrer stofflichen Zusammensetzung einen grundsätzlichen Unterschied zu jenen Extrakten, welche mit nichtalkoholischen Lösungsmitteln erhalten werden. Bei der Herstellung von Ethanolextrakten kommt es nämlich zu einer Umsetzung von freien langkettigen Carbonsäuren der Droge zu den entsprechenden Ethylestern. Diese Umsetzung tritt bei Verwendung nichtalkoholischer Lösungsmittel nicht ein.

Bei der Herstellung von Ethanolextrakten ist der Grad der Umsetzung und der Anteil der veresterten Carbonsäuren abhängig von der Drogenqualität und den genauen Prozeßparametern bei der Herstellung. Normalerweise können bei einfacher Ethanolextraktion nur Sabalestergehalte von weniger als 5 Gew.-%, bezogen auf das Gesamtgewicht des Trockenextrakts, erreicht werden. Es gibt Anhaltspunkte dafür, daß bestimmte Enzyme (Esterasen) der frischen, aber auch der getrockneten Droge katalytisch diese Veresterung beschleunigen können. Bei der Herstellung von Ethanolextrakten aus getrockneter Droge kann der Veresterungsgrad noch geringer und stärkeren Schwankungen unterworfen sein als bei der Verwendung frischer Droge. Bei lösungsmittelhaltigen ethanolischen Extrakten und Tinkturen kann es darüber hinaus im Laufe der Lagerung zu Veränderungen des Estergehaltes durch Nachveresterungen kommen, weshalb eine Standardisierung dieser Extrakte und Tinkturen bisher nur schwer möglich war.

In Z. Arzneimittelforsch. 16, S. 95 (1966) ist ein Palmetto-Öl beschrieben, das etwa 63 % freie Fettsäuren, nämlich Capron-, Capryl-, Caprin-, Laurin-, Palmitin- und Ölsäure, und etwa 37 % an Ethylestern dieser Säuren enthielt. Das beschriebene Öl wurde jedoch aus frischen Sabalfrüchten gewonnen, die mit Ethanol konserviert und vermutlich länger als 1 Jahr in Ethanol aufbewahrt worden waren. Der hohe Sabalesteranteil ist durch die lange Standzeit und die hohe Enzymaktivität der frischen Früchte zu erklären. Die industrielle Herstellung eines Extrakts mit hohem Esteranteil ist auf diese Weise nicht möglich, zumal nur die getrocknete Droge in ausreichender Menge zur Verfügung steht und entsprechend bevorratungsfähig ist.

Der Erfindung liegt deshalb die Aufgabe zugrunde, einen öligen Trockenextrakt mit hohem Gehalt (über 10 Gew.-%) an Sabalestern sowie ein in industriellem Maßstab durchführbares Verfahren zu seiner Herstellung bereitzustellen. Darüber hinaus soll ein Extrakt geschaffen werden, der diejenigen Inhaltsstoffe der Sabal-Droge enthält, denen ebenfalls ein Anteil an der pharmakologischen Wirkung zugeschrieben wird; dabei handelt es sich um unveresterte freie langkettige Carbonsäuren und Phytosterole wie Sitosterol, Sitosterolglucosid und verwandte Verbindungen.

Diese Aufgabe wird erfindungsgemäß gelöst durch einen Extrakt aus den Früchten von Sabal serrulatum, der einen Gehalt an Ethylestern der $C_{10}$ - $C_{14}$-Carbonsäuren Caprinsäure, Laurinsäure und Myristinsäure von zusammen 12 - 18 Gew.-%, vorzugsweise von 15 Gew.-%, aufweist.

Ein solcher Extrakt besitzt im Vergleich zu Extrakten, welche keine oder wesentlich geringere Mengen der genannten Ester enthalten, verbesserte antiandrogene Eigenschaften.

Vorzugsweise enthält der Extrakt zusätzlich noch einen Anteil von mindestens 6 Gew.-%, bezogen auf das Gesamtgewicht des Trockenextraktes, Ethylestern der Capron-, Capryl-, Palmitin-, Stearin- und Ölsäure. Schließlich enthält der Extrakt vorzugsweise mindestens 40 Gew.-% an freien Carbonsäuren aus der aus Capron-, Capryl-, Caprin-, Laurin-, Myristin-, Palmitin-, Stearin- und Ölsäure bestehenden Gruppe. Vorzugsweise besitzt der Extrakt einen Phytosterolgehalt von mindestens 0,1 Gew.-% und einen Gehalt an Phytosterolglucosiden von mindestens 0,05 Gew.-%.

Die benigne Prostatahyperplasie wird durch das unkontrollierte Wachstum verschiedener Zellen in der Vorsteherdrüse des alternden Mannes hervorgerufen. Als wesentliche auslösende Faktoren werden die männlichen Geschlechtshormone angesehen. Besonders Testosteron und sein Metabolit Dihydrotestosteron üben einen lebenslangen Stimulus auf die Proliferation des Prostatagewebes aus (vgl. Heinzl, Medizinische Monatsschrift für Pharmazeuten 12, S. 382 (1990)). Die Hemmung der Wirkung dieser Androgene stellt deshalb ein wirksames Prinzip zur Behandlung der benignen Prostatahyperplasie dar.

Die antiandrogene Wirkung eines sabalesterreichen lipophilen Sabalextrakts wurde im Vergleich zu einem lipophilen Sabalesterextrakt mit weniger als 5 % Sabalestern bei kastrierten Ratten nach exogenem Ersatz von Testosteron untersucht (vgl. Hebborn, Screening Methods in Pharmacology, Vol. II, 75, 1971). Männliche Ratten (Sprague-Dawley, Interfauna, Tuttlingen) mit einem Körpergewicht von etwa 90-100 g wurden unter Anästhesie mit Xylazin (7 mg/kg i.p.) und Ketamin (66 mg/kg i.p.) kastriert. Der Ersatz von endogenem Testosteron erfolgte durch tägliche subkutane Injektionen von 250 $\mu$g/kg Testosteronpropionat (TP) in 1 ml Olivenöl. Gleichzeitig mit der Testosteronapplikation wurden die Versuchstiere mit 1 oder 0,3 ml/kg Sabalextrakt (SE) (Trockenextrakt, gewonnen a) erfindungsgemäß durch Extraktion der Droge mit Ethanol bzw. b) durch Extraktion mit Hexan) in 2,5 ml/kg Olivenöl peroral behandelt. Nach sechstägiger Behandlung wurden die Tiere am folgenden Tag in Äthernarkose getötet und das Gewicht der ventralen Prostata sowie der Glandula vesicularis einschließlich der Koagulationsdrüse ermittelt.

Wie aus den Daten der Tabelle 1 ersichtlich ist, besitzt der sabalesterreiche Sabalextrakt in den beiden verwendeten Dosen eine ausgeprägte antiandrogene Wirkung, die in einer signifikanten Reduktion der Größe der akzessorischen Geschlechtsorgane zum Ausdruck kommt. Für den lipophilen Sabalesterextrakt mit weniger als 5 % Sabalestern war bei den gleichen Dosen kein Einfluß auf das Gewicht der Prostata und der Glandula vesicularis nachweisbar. Die Ergebnisse machen deutlich, daß die Fettsäureester maßgeblich an den antiandrogenen Eigenschaften eines alkoholischen Sabalextraktes beteiligt sind.

Weitere Untersuchungen haben gezeigt, daß die verbesserte antiandrogene Eigenschaft des sabalesterreichen Sabal-Extraktes unter anderem auf der verbesserten Bioverfügbarkeit der Ester im Vergleich zu den freien Fettsäuren beruht. In Tabelle 2 ist die gemessene Radioaktivität in Gewebe von ventraler Prostata, Seminal-Vesikel (einschließlich der Koagulationsdrüsen) und Plasma bei Ratten nach Gabe von 300 mg/kg des erfindungsgemäß hergestellten ethanolischen Extraktes, dotiert mit jeweils 5 $\mu$Ci $^{14}$C-Laurinsäure oder $^{14}$C-Laurinsäureethylester angegeben. Eine Stunde nach Gabe wird im Plasma der Tiere, die den Ester erhielten, signifikant mehr Radioaktivität gemessen als bei den Tieren, die die Säure erhielten. Zwei Stunden nach Gabe enthalten Prostata, Seminal-Vesikel und Plasma der Tiere, die den Ester erhielten, signifikant mehr Radioaktivität, im Vergleich zur Gruppe der Tiere, die die Säure erhielten.

Zur Herstellung des erfindungsgemäßen Extraktes wird folgendermaßen verfahren:

Obwohl frische und/oder getrocknete Sabalfrüchte verwendet werden können, werden aus Gründen der Standardisierbarkeit und der industriellen Durchführbarkeit des Verfahrens getrocknete Früchte als Ausgangsmaterial bevorzugt. Die getrockneten Sabalfrüchte werden mit Ethanol mazeriert. Hierbei kommt es zur Esterbildung, wobei die Geschwindigkeit und das Ausmaß der Veresterung abhängen von der Mazerationszeit, dem Reifegrad der verwendeten Früchte, der Enzymaktivität des Drogenmaterials, dem Gehalt der Droge an Carbonsäuren und dem genauen pH-Wert des Mazerats. Durch Steuerung dieser Parameter durch kontinuierliche Messung der IST-Werte und Vergleich mit den gewünschten bzw. empirisch gewonnenen SOLL-Werten und durch laufende Überwachung des Veresterungsgrades werden Estergehalte von 12 - 18 Gew.-%, bezogen auf den Trockenextrakt, eingestellt. Durch Vermischen einzelner Ansätze wird auf einen Sollwert von 15 % Sabalester standardisiert. Der Trockenextrakt wird durch Entfernen des Lösungsmittels und durch Abtennung hydrophiler Bestandteile über einen Phasentrenner als Öl erhalten.

## Beispiel

1500 kg trockene, geschrotete Sabalbeeren werden in einer Mazerationsanlage mit 3000 kg vergälltem Ethanol (> 90%) versetzt. Im Laufe der Mazeration wird in regelmäßigen Abständen auf Trockenextraktausbeute, Sabalestergehalt und pH-Wert geprüft. Die Mazeration wird bis zur Erzielung eines Estergehalts von zwischen 12 und 18 % fortgesetzt.

In der Regel kommt es zu einem Stillstand der Spontanveresterung mit einem Ansteigen des pH-Wertes von ursprünglich 3 auf Werte um 5. Dann ist bei einem Sabalestergehalt von üblicherweise weniger als 5 Gew.-% eine pH-Korrektur auf den Ausgangswert vorzunehmen, was zu einer erneuten Aktivierung der Esterbildung führt. Die Korrektur des pH-Werts wird auf möglichst schonende Weise durchgeführt; vorzugsweise wird das Mazerat über einen sauren Ionenaustauscher gepumpt und dadurch auf einen pH-Wert von etwa 3 eingestellt.

Die Extraktlösung wird anschließend auf einen öligen Trockenextraktanteil von mehr als 90 % eingeengt. Hydrophile Bestandteile werden vom lipophilen Ölextrakt über einen Phasentrenner abgeschieden. Das Sabalöl wird filtriert. Verschiedene Chargen des Sabalextrakts werden durch Vermischen auf einen Gehalt von 15 Gew.-% Sabalestern standardisiert. Die Ausbeuten betragen etwa 8 %, bezogen auf die eingesetzte Drogenmenge.

Der erfindungsgemäße Extrakt wird vorzugsweise zur Herstellung von Arzneimitteln, insbesondere von Phytopharmaka verwendet; er kann auch als solcher, gegebenenfalls auch zusammen mit üblichen pharmazeutischen Hilfs- und Zusatzstoffen, als Antiandrogenum und Antiexsudativum verwendet werden, auch in Kombination mit anderen Phytotherapeutika.

Gegenstand der Erfindung sind somit auch Arzneimittel, die den erfindungsgemäßen Extrakt sowie gegebenenfalls übliche Hilfs- und und Zusatzstoffe enthalten.

Tabelle 1: Antiandrogene Wirkung von Sabalextrakten in kastrierten Ratten

| | Normale Ratten | Kastrierte Ratten | Kastrierte Ratten + TP + Olivenöl | Kastrierte Ratten + TP + 1 ml/kg SE | Kastrierte Ratten + TP + 0,3 ml/kg SE |
|---|---|---|---|---|---|
| **a) Ethanolextrakt (15 % Sabalester)** | | | | | |
| Tierzahl | 9 | 9 | 9 | 9 | 9 |
| Körpergewicht (g) | 149 ± 3,8 | 145 ± 2,8 | 151 ± 4,0 | 137 ± 4,1 | 133 ± 3,4 |
| Ventrale Prostata (mg) | 90,5 ± 5,6 | 14,7 ± 0,7 | 82,1 ± 5,7 | 66,5 ± 5,8 (-19 %)° | 65,7 ± 4,7* (-20 %)° |
| Gl. vesicularis einschl. Koagulationsdrüse (mg) | 94,3 ± 7,6 | 28,4 ± 1,1 | 96,1 ± 5,4 | 79,2 ± 4,0* (- 18 %)° | 69,3 ± 2,4* (- 28 %)° |
| **b) Hexanextrakt (< 5 % Sabalester)** | | | | | |
| Tierzahl | 10 | 10 | 10 | 10 | 10 |
| Körpergewicht (g) | 135 ± 3,7 | 132 ± 3,2 | 137 ± 2,9 | 129 ± 3,6 | 139 ± 2,7 |
| Ventrale Prostata (mg) | 62,6 ± 4,9 | 14,0 ± 0,5 | 77,7 ± 2,0 | 77,3 ± 2,5 (- 0,5 %)° | 82,6 ± 4,8 (+ 6 %)° |
| Gl. vesicularis einschl. Koagulationsdrüse (mg) | 59,3 ± 4,1 | 23,6 ± 0,7 | 102,8 ± 3,7 | 99,4 ± 4,0 (- 3 %)° | 102,0 ± 3,2 (- 1 %)° |

Angegeben sind die Mittelwerte und der Standardfehler.
° Verringerung des Gewichts (in Prozent) gegenüber den kastrierten Ratten, die nur Testosteronpropionat und Olivenöl erhielten.
* statistisch signifikant (p < 0,05)

## Tabelle 2

Bioverfügbarkeit von Sabalestern im Vergleich zur Bioverfügbarkeit von freien Fettsäuren

| Zeit nach Gabe | Organ | Radioaktivität [DPM/g] | | n |
|---|---|---|---|---|
| | | Ester | Säure | |
| 1 h | Prostata | $3504 \pm 2314$ | $2786 \pm 834$ | 3 |
| | Vesikel | $2526 \pm 1068$ | $1400 \pm 1412$ | 3 |
| | Plasma | $8539 \pm 3137*$ | $2273 \pm 942$ | 3 |
| 2 h | Prostata | $9146 \pm 2175*$ | $2304 \pm 537$ | 3 |
| | Vesikel | $4227 \pm 693**$ | $1641 \pm 588$ | 3 |
| | Plasma | $13852 \pm 1564***$ | $1710 \pm 1304$ | 3 |

Angegeben sind Mittelwert $\pm$ Standardabweichung

Signifikanzniveau    * $p < 0,05$    DPM = decays per minute

                                  ** $p < 0,01$    n = Zahl der Versuchstiere

                            *** $p < 0,001$

## Patentansprüche

1. Öliger Trockenextrakt aus den Früchten von Sabal serrulatum (Serenoa repens), gekennzeichnet durch einen Gehalt an Ethylestern der Caprin-, Laurin- und Myristinsäure von zusammen 12 - 18 Gew.-%.

2. Standardisierter Extrakt nach Anspruch 1, gekennzeichnet durch einen Gehalt an Ethylestern der Caprin-, Laurin- und Myristinsäure von zusammen 15 Gew.-%.

3. Extrakt nach Anspruch 1 oder 2, gekennzeichnet durch einen weiteren Anteil von mindestens 6 Gew.-%, bezogen auf das Gesamtgewicht des Trockenextraktes, Ethylestern der Capron-, Capryl-, Palmitin-, Stearin- und Ölsäure.

4. Extrakt nach einem der Ansprüche 1 bis 3, gekennzeichnet durch einen Gehalt von mindestens 40 Gew.-% an freien Carbonsäuren aus der aus Capron-, Capryl-, Caprin-, Laurin-, Myristin-, Palmitin-, Stearin- und Ölsäure bestehenden Gruppe.

5. Extrakt nach einem der Ansprüche 1 bis 4, gekennzeichnet durch einen Phytosterolgehalt von mindestens 0,1 Gew.-% und einen Gehalt an Phytosterolglucosiden von mindestens 0,05 Gew.-%.

6. Extrakt nach einem der Ansprüche 1 bis 5, dadurch gekennzeichnet, daß er aus getrockneten Früchten von Sabal serrulatum gewonnen ist.

7. Verfahren zur Herstellung des Sabalextrakts gemäß einem der Ansprüche 1 bis 6, bei dem Früchte von Sabal serrulatum (Serenoa repens) mit Ethanol mazeriert werden und nach dem Abziehen des Lösungsmittels vom Mazerat hydrophile Bestandteile über einen Phasentrenner abgetrennt werden, dadurch gekennzeichnet, daß der Sabalestergehalt und der pH-Wert des Mazerats ständig gemessen und mit den Soll-Werten verglichen werden und daß der pH-Wert solange auf etwa 3 gehalten wird, bis der Sabalestergehalt, bezogen auf den Trockenextrakt, 12 bis 18 Gew.-% beträgt.

8. Verfahren nach Anspruch 7, dadurch gekennzeichnet, daß verschiedene Chargen des Sabalextraktes miteinander vermischt und dadurch auf einen Gehalt von 15 Gew.-% Sabalestern standardisiert werden.

9. Verfahren nach Anspruch 7 oder 8, dadurch gekennzeichnet, daß nur getrocknete Früchte von Sabal serrulatum mazeriert werden.

10. Verwendung des Trockenextraktes gemäß einem der Ansprüche 1 bis 6 zur Herstellung von Arzneimitteln, insbesondere von Phytopharmaka.

11. Antiandrogen und antiexsudativ wirksames Arzneimittel, enthaltend einen Extrakt gemäß einem der Ansprüche 1 bis 6 sowie gegebenenfalls übliche Hilfs- und Zusatzstoffe.

| EINSCHLÄGIGE DOKUMENTE | | | EP 91121348.6 | |
|---|---|---|---|---|
| Kategorie | Kennzeichnung des Dokuments mit Angabe, soweit erforderlich, der maßgeblichen Teile | | Betrifft Anspruch | KLASSIFIKATION DER ANMELDUNG (Int Cl⁵) |
| D,X | ARZNEIMITTELFORSCHUNG, 16. Jahrgang, Heft 1, 1966, AULENDORF/WÜRTT., P. KLOSS "Die wasserdampf-flüchtigen Bestandteile des Preßsaftes von Sabal serru-latum (Reem et Schult)" Seiten 95,96 * Seite 95 * -- | | 1-4, 10,11 | A 61 K 35/78 |
| X | HAGERS HANDBUCH DER PAHRMA-ZEUTISCHEN PRAXIS, Band 6, 1979, Springer Verlag, Berlin-Heidelberg-New York, P.H. LIST et al. "Sera und Impfstoffe" * Seiten 370,371 * -- | | 1-5, 10,11 | |
| A | EP - A - 0 287 000 (CONSIGLIO NAZIONALE DELLE RICERCHE) * Seiten 2,3 * -- | | 1-6, 10,11 | RECHERCHIERTE SACHGEBIETE (Int Cl⁵) |
| A | FR - A - 2 480 754 (P. FABRE SA.) * Patentansprüche; Seite 1, Zeilen 14-21 * ---- | | 1-11 | A 61 K 35/00 |
| | Der vorliegende Recherchenbericht wurde für alle Patentansprüche erstellt. | | | |

| Recherchenort | Abschlußdatum der Recherche | Prüfer |
|---|---|---|
| WIEN | 06-04-1992 | IRMLER |